(19) Europäisches Patentamt
European Patent Office
Office européen des brevets

(11) Publication number: 0 248 954 B1

(12) # EUROPEAN PATENT SPECIFICATION

(45) Date of publication of patent specification: 01.04.92

(51) Int. Cl.5: C07C 33/46, C07C 29/86, A01N 29/12

(21) Application number: 86304437.6

(22) Date of filing: 10.06.86

Divisional application 91306349.1 filed on 10/06/86.

(54) **Process for reducing the amount of DDT-related impurities in 1,1-bis(chlorophenyl)-2,2,2-trichloro-ethanol.**

(43) Date of publication of application:
**16.12.87 Bulletin 87/51**

(45) Publication of the grant of the patent:
**01.04.92 Bulletin 92/14**

(84) Designated Contracting States:
**AT BE CH DE FR GB LI NL**

(56) References cited:
**SU-A- 136 346**
**US-A- 2 812 362**

(73) Proprietor: **ROHM AND HAAS COMPANY**
**Independence Mall West**
**Philadelphia Pennsylvania 19105(US)**

(72) Inventor: **Nichols, Richard Walter**
**102 Glenwood Drive**
**Washington Crossing, PA 18977(US)**
Inventor: **Zabrodski, William Josepf**
**1191 Highland Avenue**
**Bensalem PA 19020(US)**
Inventor: **Mitra, Abhijit**
**1989 Dogwood Drive**
**Westfield NJ 07090(US)**

(74) Representative: **Angell, David Whilton et al**
**ROHM AND HAAS (UK) LTD. European Operations Patent Department Lennig House 2 Mason's Avenue**
**Croydon CR9 3NB(GB)**

EP 0 248 954 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention is concerned with a process for preparing 1,1-bis(chlorophenyl)-2,2-trichloroethanol of high purity in relation to DDT-related impurities.

The 1,1-bis(halophenyl)-2,2,2-trichloroethanols are miticidal agents, the preparation of which is described in U.S. Patents Nos. 2,812,280 and 2,812,362. 1,1-Bis(chlorophenyl)-2,2,2-trichloroethanol (dicofol) is a commercial miticide and there are various pesticidal compositions which use it as an active ingredient.

The present commercial process for manufacturing 1,1-bis(chlorophenyl)-2,2,2-trichloroethanol (dicofol) uses 1,1-bis(chlorophenyl)-2,2,2-trichloroethane (DDT) as the starting material. DDT is dehydrohalogenated with alkali to afford 1,1-bis(chlorophenyl)dichloroethylene, which is chlorinated to afford 1,1-bis-(chlorophenyl)-1,2,2,2,-tetrachloroethane.

The 1,1-bis(chlorophenyl)-1,2,2,2-tetrachloroethane is then converted to the desired product, dicofol, by placing it in a hydrolysis reaction vessel with p-toluenesulfonic acid, sulfuric acid (99%) and water and then heating to about 110°C. Steam is injected into the reactor while continuing to heat the batch to about 140°C. Distillate from the reactor is monitored to ascertain an end point specific gravity of about 1.100 at 20°C. When the batch reaches about 140°C and the specific gravity requirement is met, the batch continues to be heated to from about 143°C to about 145°C and water, in addition to steam, is added. The condensate continues to be monitored to an end point specific gravity to about 1.04 at 20°C. The batch is cooled to about 130°C, transferred to a hold tank, and allowed to settle for about 2 hours. The lower dicofol technical layer is separated and washed three times from about 95°C to about 100°C using about 0.3 pounds (1b.) of water per pound of dicofol technical. The batch is dehydrated at about 105°C and 26 inches Hg vacuum to afford the desired dicofol technical grade.

Technical dicofol made by the present commercial process typically contains from about 2% to about 8% DDT related impurities (DDTr's).

The effectiveness of 1,1-bis(chlorophenyl)-2,2,2-trichloroethanol (dicofol) as an acaricide has encouraged extensive use of dicofol and compositions containing dicofol in agriculture and horticulture.

The DDTr's which are encountered in the present commercial process used to manufacture dicofol include: 1,1-bis-(4-chlorophenyl)-2,2,2-trichloroethane (p,p′-DDT); 1-(4-chlorophenyl)-1-(2-chlorophenyl)-2,2,2-trichloroethane (o,p′-DDT); 1,1,-bis-(4-chlorophenyl)-2,2-dichloroethylene (p,p′-DDE); 1-(4-chlorophenyl-1-(2-chlorophenyl)-2,2-dichloroethylene (o,p′-DDE); 1,1-bis-(4-chlorophenyl)-2,2-dichloroethane (p,p′-DDD); 1-(4-chlorophenyl)-1-(2-chlorophenyl)-2,2-dichloroethane (o,p′-DDD); 1,1-bis-(4-chlorophenyl)-1,2,2,2-tetrachloroethane (p,p′-Cl-DDT); 1-(4-chlorophenyl-1-(2-chlorophenyl)-1,2,2,2-tetrachloroethane (o,p′-Cl-DDT). Non-DDTr impurities which are encountered in the present commercial process used to manufacture dicofol include: 4,4′-dichlorobenzophenone (p,p′-DCBP); 2,4′-dichlorobenzophenone (o,p′-DCBP); 4,4′-dichlorobenzil (p,p′-DCBZ); and 2,4-dichlorobenzil (o,p′-DCBZ). DDTr's are present along with the desired dicofol product due to the impurity of DDT used as a starting reactant and formation during the conversion of DDT to dicofol. As a group, DDTr's are suspected of posing an environmental risk. Accordingly, it is desirable to find means to eliminate or reduce these DDTr impurities.

This invention enables the preparation of 1,1-bis(chlorophenyl)-2,2,2-trichloroethanol substantially free of DDTr's by reducing the amount of these impurities in dicofol containing them.

Unless otherwise stated, all parts and percentages used herein are by weight.

In accordance with the present invention, there is provided a practical process for producing "DDTr free" (as herein defined) 1,1-bis-(chlorophenyl)-2,2,2-trichloroethanol by selective solvent extraction which comprises at a temperature of from about -5°C to about 100°C:

(a) dissolving technical 1,1-bis(chlorophenyl)-2,2,2-trichloroethanol in water-miscible solvent, particularly hydroxylic solvent such as one or more of the following: ($C_1$-$C_3$) alcohols, ($C_2$-$C_6$) diols, ($C_1$-$C_3$) organic acids, 4-hydroxy-3-methyl-2-butanone, or solvent comprising water and non-hydroxylic solvent capable of affording hydroxylic solvent when mixed with water, such as acetone, the solution containing 0 to 50% water based on the amount of non-aqueous solvent, to obtain a solution having a 1,1-bis(chlorophenyl)-2,2,2-trichloroethanol concentration of from about 1% to about 60%; and

(b) extracting the solution with a straight, branched or cyclized ($C_4$-$C_{20}$)alkane; straight, branched or cyclized ($C_4$-$C_{20}$)alkane; straight, branched or cyclized ($C_6$-$C_{20}$)alkadiene; ($C_6$-$C_{10}$) single ring aromatic; or a mixture thereof; and

(c) separating the resulting phases; and

(d) removing the solvent from the dicofol-containing phase, to yield dicofol of the desired purity.

The following diagram indicates the starting materials, intermediates, DDT related impurities and final product of the present process for preparing dicofol.

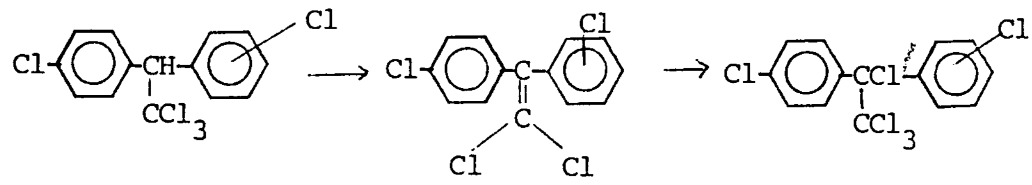

DDT

DDE

Cl - DDT

p,p'-DDT,73-77%
o,p'-DDT, 19-22%
p,p'- and o,p'-DDD, 0.2-0.4%
p,p'- and o,p'-DDE, 0.5 - 2%

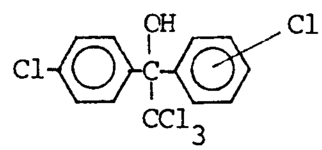

Dicofol

p,p'- and o,p'-DDE, 0.5-2%
p,p'- and o,p'-Cl-DDE 1-6%
p,p'- and o,p'-DDD, <0.01%
p,p'- and o,p'-DDT, <0.01%

The DDt used as a starting material typically contains from about 73% to about 77% p,p′-DDT; from about 19% to about 22% o,p′-DDT; and from about 0.2 to about 0.4% p,p′- and o,p′-DDD.

The conversion of DDT to DDE is an efficient reaction and typically the dicofol produced will contain less than 0.01% DDT. However, non-ideal reaction conditions can result in higher levels of DDT-contaminated DDE and, consequently, result in higher levels of DDT in the dicofol product.

In practice, conversion of DDE to Cl-DDT of greater than 99% is difficult to achieve. Therefore, Cl-DDT typically contains from about 0.5% to about 2% DDE. Further, since chlorination of o,p′-DDE to form o,p-Cl-DDT is significantly slower than chlorination of p,p′-DDE to form p,p′-Cl-DDT (approximately 50 times slower) the bulk of the residual DDE in the Cl-DDT produced is o,p′-DDE. Dicofol technical typically contains from about 0.5% to about 2% DDE, both o,p′-DDE.

In the hydrolysis of Cl-DDT to dicofol, a point is reached beyond which further conversion of Cl-DDT becomes very slow. This is believed to be due to a mode of dicofol degradation that yields Cl-DDT. As the above-mentioned point is approached, other dicofol degradation products accumulate in the batch. These degradation products include DCBP and DCBZ. Dicofol technical typically contains o,p′-Cl-DDT and p,p′-Cl-DDT at levels of from about 1% to about 6%.

It has now been discovered that DDTr's can be efficiently removed from dicofol technical by "selective solvent extraction".

Dicofol free of DDTr's, that is, containing less than 0.1% of DDTr's, can be prepared by the present invention.

"Selective solvent extraction" means unequal distribution of dicofol and DDTr's between two particular liquid phases to prepare DDTr free dicofol. Generally, this extraction may be accomplished by continuous liquid-liquid countercurrent extraction or by sequential batch extraction.

For economic reasons, where DDTr's are present at from about 1% or higher, this extraction may preferably be accomplished by means of continuous liquid-liquid countercurrent extraction; where DDTr levels are below about 1%, then this extraction may preferably be accomplished by means of batch extraction.

It has been found that DDTr removal from dicofol technical may be accomplished by employing limited classes of solvents. Hydrocarbons in general and in particular straight, branched or cyclized ($C_4$-$C_{20}$)-alkanes such as hexane, heptane, octane, isooctane, cyclohexane and the like; straight, branched or

cyclized ($C_4$-$C_{20}$)alkenes such as diisobutylene, cyclohexene and the like; straight, branched or cyclized ($C_6$-$C_{20}$) cumulated, conjugated or isolated alkadienes such as dicyclopentadiene and the like; ($C_6$-$C_{10}$) single ring aromatics such as benzene, toluene, xylene, cumene and the like; or mixtures or said solvents may be used as one of the liquid phases ("extracting solvent phase") in selective solvent extraction. The second liquid phase ("raffinate phase") is limited to water miscible solvents including but not limited to hydroxylic solvents such as ($C_1$-$C_3$) alcohols, for example, methanol and ethanol and the like; ($C_2$-$C_6$) diols, for example, ethylene glycol, 1,3-butanediol, 2,5-hexanediol and the like; ($C_1$-$C_3$) organic acids, for example, acetic acid, formic acid and the like; 4-hydroxy-3-methyl-2-butanone; or a solvent capable of affording a hydroxylic solvent when mixed with water, for example, acetone and the like; or mixtures of the above solvents. Typically, the raffinate phase will be the lower layer, although with particular solvents, it may be the upper layer.

Separation between the two phases is aided by the presence of water. The actual amount of water used varies depending upon the selection of solvents and concentration of dicofol technical. Generally, up to about 50% water may be used. The precise amount of preferred water used with a given pair of solvents (raffinate phase solvent and extracting phase solvent) can readily be determined by one skilled in the art. We believe that the presence of water, while preferred when a diol is used as the raffinate phase solvent, will be regarded as very highly preferred for efficient operation when the other hydroxylic raffinate phase solvents are used.

Temperatures in the range of from about -5° C to about 100° C may be used. The actual temperature employed will depend on the selection of solvents and will be apparent to one skilled in the art after making the selection of solvents for the two liquid phases.

Depending on the selection of solvents, it may be necessary to employ pressure during extraction. The amount of pressure required will depend on the selection of solvents and will be apparent to one skilled in the art.

Dicofol technical is dissolved in the, optionally aqueous, solvent(s) (raffinate phase) preparatory to the extraction of DDTr's. The dicofol concentration in such solutions will generally be from about 1% to about 60%.

Preferably, the extracting solvent phase will comprise ($C_5$-$C_{16}$)alkane(s) or ($C_5$-$C_{15}$)alkene(s) or mixtures thereof and most preferably mixed heptanes having a boiling point in the range of from about 91° to about 97° C.

Preferably, the raffinate phase will comprise aqueous methanol (87-93%); aqueous ethanol (70-85%); or ($C_2$-$C_6$) diol(s) with 0-10% water and the most preferable being aqueous methanol.

In the case of methanol specifically, the amount of water employed will most preferably be in the range of from about 9% to about 13%.

Preferably, the temperature employed is between from about 10° C to about 60° C and, most preferably, between from about 20° C to about 40° C.

Preferably the dicofol concentration in the solution in step (a) is between from about 10% to about 50% and, most preferably, between from about 25% to about 35%.

Extraction of DDTr's from dicofol technical using n-haptane and 90% methanol- 10% water (90% aqueous methanol)

The results of this experiment are set forth below in Tables I, II and III. Table I shows the physical data of the system (volume, weight and concentrated weight). Table II shows the reported analytical results. Table III shows the calculated results, based on the analytical data, for recovery of dicofol and the percentage of DDTr's removed. The terms "partition coefficient" and "distribution coefficient" are used interchangeably herein.

A 50g sample of the desired dicofol solution was taken in a stoppered 100 ml graduated cylinder. The initial volumes were recorded (Table I - Column 1). n-Heptane (20g.) was added to all samples and stoppered. They were then shaken well for two minutes. The samples were placed in an ice-water bath for 30 minutes. After the cooling period all samples were again shaken for 2 minutes. The samples were returned to the bath and allowed to equilibrate for 30 minutes. The samples were then removed from the bath and volumes recorded (Table I - Columns 2 and 3). The layers were then separated and each layer weighed (Columns 4 and 5). The separated layers were concentrated under vacuum (70° C, 1 mm Hg), weighed (columns 6 and 7) and analyzed (Table II).

The results (Table III) demonstrate that the DDTr fraction was preferentially extracted by n-heptane.

Table I

Partition of Dicofol Technical Between n-Heptane and 90% Aqueous Methanol at 0°C[1]

| Dicofol Solution (Volume) | Heptane Layer Volume | Methanol Layer Volume | Heptane Layer Weight | Methanol Layer Weight | Heptane Layer Weight (Stripped) | Methanol Layer Weight (Stripped) |
|---|---|---|---|---|---|---|
| 50% (47.0 ml) | 22 ml | 53 ml | 16.29 g | 52.68 g | 1.72 g | 22.77 g |
| 33% (52.0 ml) | 24.5 ml | 55.5 ml | 17.62 g | 51.38 g | 1.44 g | 14.81 g |
| 16.7% (57.5 ml) | 25.5 ml | 59.5 ml | 18.18 g | 51.48 g | 0.91 g | 7.43 g |
| 8.4% (59.5 ml) | 26.5 ml | 61.0 ml | 18.19 g | 51.44 g | 0.46 g | 3.66 g |
| 4.2% (60.5 ml) | 26.5 ml | 62.0 ml | 17.94 g | 51.21 g | 0.24 g | 1.79 g |

[1] 20 g (29.4 ml) n-heptane and 50 g dicofol solution were used.

Table II

Analytical Results

| | Dicofol Solution | OP' DCBP | OP' DCBZ | PP' DCBP | PP' DCBZ | OP' Dicofol | PP' Dicofol | OP' DDE | PP' DDE | OP' Cl-DDT | PP' Cl-DDT | % DDTR's | % Account-ability |
|---|---|---|---|---|---|---|---|---|---|---|---|---|---|
| A | | | | | | | | | | | | | |
| Heptane layer | 50% | 1.5 | 1.7 | 1.7 | 0.6 | 7.1 | 51.7 | 4.8 | 1.0 | 0.4 | 3.4 | 9.6 | 81.0 |
| | 33% | 1.5 | 1.8 | 1.5 | 0.7 | 6.0 | 47.3 | 5.2 | 1.6 | 1.2 | 3.4 | 11.4 | 77.0 |
| | 16.7% | 1.5 | 1.9 | 1.6 | 0.9 | 5.3 | 45.6 | 5.5 | 1.4 | 1.1 | 4.0 | 12.0 | 76.1 |
| | 8.4% | 1.6 | 2.0 | 1.5 | 1.0 | 5.5 | 47.1 | 6.1 | 1.3 | 0.7 | 5.1 | 13.2 | 79.7 |
| | 4.2% | 1.9 | 2.4 | 1.6 | 1.7 | 5.3 | 48.0 | 6.2 | 1.2 | 0.8 | 5.5 | 13.7 | 83.0 |
| | Starting Dicofol | 1.0 | 1.5 | 0.6 | 0.33 | 13.0 | 74.6 | 1.1 | 0.2 | 0.1 | 0.9 | 2.3 | 96.0 |
| B | | | | | | | | | | | | | |
| Agueous Methanol layer | 50% | 1.8 | 1.1 | 1.3 | 0.5 | 12.9 | 74.2 | 0.9 | 0.1 | 0.1 | 0.5 | 1.6 | 94.7 |
| | 33% | 1.7 | 1.2 | 1.3 | 0.3 | 12.8 | 73.4 | 0.8 | 0.1 | 0.1 | 0.4 | 1.4 | 93.8 |
| | 16.7% | 1.6 | 1.0 | 1.5 | 0.5 | 13.3 | 76.3 | 0.6 | 0.1 | 0.1 | 0.3 | 1.1 | 97.1 |
| | 8.4% | 1.6 | 1.0 | 1.4 | 0.4 | 13.2 | 76.4 | 0.6 | 0.1 | 0.1 | 0.2 | 1.1 | 96.9 |
| | 4.2% | 1.8 | 0.9 | 1.2 | 0.4 | 13.8 | 78.2 | 0.5 | .05 | 0.3 | 0.2 | 1.0 | 99.0 |

Table III

| Calculated Results from 90% Aqueous Methanol Solution, 0 °C | | | | |
|---|---|---|---|---|
| Dicofol Solutions | Recovered Weight (%) | % DDTr's Removed | % p,p'-Dicofol Recovered | % o,p'-Dicofol Recovered |
| 50% | 93.0 | 31 | 95.0 | 96.0 |
| 33% | 91.1 | 45 | 94.1 | 95.6 |
| 16.7% | 89.1 | 57 | 93.2 | 95.3 |
| 8.4% | 88.8 | 60 | 92.8 | 95.0 |
| 4.2% | 88.2 | 65 | 92.4 | 95.1 |

In subsequent experiments, the partitioning of dicofol technical between n-heptane and 90% aqueous methanol was also evaluated at 23 °C. The combined results from the 0 °C and 23 °C experiments are presented in Table IV where the results are expressed as distribution coefficients for dicofol and the two principal DDTr's found in dicofol technical, o,p'-DDE and p,p'-Cl-DDT. In all cases, the extractability of DDTr's exceeded that of dicofol by a significant margin.

Table IV

| Distribution Coefficients[1] and Selectivities[2] from Extraction Experiments | | | | | | | |
|---|---|---|---|---|---|---|---|
| Conc. of Aq.[3] Dicofol Sol'n | Heptane (grams) | Temp. °C | o,p' and p,p' Dicofol[4] | o,p' DDE | | p,p'-Cl-DDT | |
| | | | D.C. | D.C. | Sel. | D.C. | Sel. |
| 50 | 20 | 0 | .16 | 1.3 | 7.9 | 1.5 | 9.4 |
| | 40 | 23 | .31 | 1.66 | 5.3 | 2.1 | 6.8 |
| 40 | 40 | 23 | .32 | 2.32 | 7.2 | 2.8 | 8.6 |
| 33 | 20 | 0 | .17 | 1.8 | 10.5 | 2.6 | 14.9 |
| 30 | 40 | 23 | .30 | 2.4 | 7.9 | 4.0 | 13.1 |
| 20 | 40 | 23 | .31 | 3.5 | 11.0 | 5.3 | 16.8 |
| 16.7 | 20 | 0 | .20 | 3.2 | 16.1 | 4.4 | 22.4 |
| | 20 | 23 | .33 | 3.4 | 10.1 | 4.1 | 12.2 |
| 10 | 40 | 23 | .32 | 3.9 | 12.2 | 7.5 | 23.4 |
| 8.4 | 20 | 0 | .21 | 3.6 | 17.3 | 9.1 | 23.3 |

[1] Distribution coefficient (D.C.): grams of solute per gram of extract layer (heptane) divided by grams of solute per gram of raffinate layer.
[2] Selectivity (Sel.): Ratio of DDTr distribution coefficient to that of dicofol.
[3] All dicofol solutions made up with 90% aqueous methanol; 50g of solution used in each extraction.
[4] The distribution coefficients of o,p' and p,p' dicofol are essentially the same.

Another series of extraction experiments was done to demonstrate that p,p'-DDT and p,p'-DDD, DDTr's, not normally present in significant amounts in dicofol, could be removed by the extraction approach. These experiments are summarized in Table V, which shows that p,p'-DDT is very preferentially extracted by n-heptane and p,p'-DDD, while not so easily extracted, is also preferentially removed.

Table V

| Distribution Coefficients for p,p'-DDT and p,p'-DDD | | | | | | | | | | |
|---|---|---|---|---|---|---|---|---|---|
| % DDD,[1,2] DDT(Wt %) | D.C. Dicofol | o.p'-DDE | | p,p'-ClDDT | | p,p'-DDD | | p,p'-DDT | |
| | | D.C. | Sel. | D.C. | Sel. | D.C. | Sel. | D.C. | Sel. |
| 0.25 | .31 | 2.4 | 7.6 | 3.7 | 11.8 | 0.66 | 2.1 | 1.9 | 5.9 |
| 0.72 | .33 | 2.4 | 7.2 | 3.8 | 11.4 | 0.71 | 2.1 | 1.9 | 5.6 |
| 1.80 | .35 | 2.4 | 6.8 | 3.8 | 10.8 | 0.79 | 2.2 | 2.1 | 6.0 |

[1] As a percentage of dicofol technical. Extractions used 50g of 33% solution of dicofol technical in 90% aqueous methanol contacted with 40g of n-heptane at 23° C.
[2] Dicofol technical containing usual quantities of o,p'-DDE and p,p'-Cl-DDT was spiked with three levels of p,p'-DDD and separately with three levels of p,p'-DDT.

The effect on distribution coefficients and selectivities of the amount of water in the methanol was determined. Results of this study are presented in Table VI. As can be seen, the percentage of water used has an effect on all monitored materials in the system. All distribution coefficients rise as the percentage of water increases. The selectivities rise initially but then begin to decrease at water levels higher than a maximum range. Importantly, if the water in the methanol is omitted, the addition of practical concentrations of dicofol technical causes the phases to coalesce.

Table VI

| Effect of Water Content on Distribution Coefficients and Selectivities | | | | | |
|---|---|---|---|---|---|
| % $H_2O$ in Aq. MeOH[1] | D.C. Dicofol | o,p'-DDE | | p,p'-Cl-DDT | |
| | | D.C. | Sel. | D.C. | Sel. |
| 4 | .27 | 1.5 | 5.7 | 1.9 | 7.0 |
| 8 | .29 | 2.2 | 7.7 | 2.9 | 10.1 |
| 12 | .38 | 3.1 | 8.3 | 4.6 | 12.2 |
| 16 | .60 | 3.6 | 6.1 | 6.8 | 11.4 |

[1] Single stage extractions conducted by contacting 50g of 33% solution of dicofol technical in various aqueous methanols with 40 g of n-heptane at 23° C.

Alternative solvent systems were evaluated. The distribution coefficients were determined by partitioning dicofol technical.

In Table VII, n-heptane was used as the extractant and different raffinate phase solvents were evaluated. In Table VIII, ethanol was used as the raffinate solvent and different extracting solvents were evaluated. In Table IX, n-heptane was used as the extractant and ethanol at different aqueous concentrations was used as the raffinate solvent. In the experiments set forth in Table IX, 30% dicofol technical solution (in the raffinate layer) was used and 50 grams of this solution was partitioned with 40 grams of the extracting solvent. In Table X, methanol was used as the raffinate solvent and different extracting solvents were evaluated. And in Table XI, using n-heptane as the extracting solvent and aqueous methanol as the raffinate solvent, the partition coefficients were determined as a function of temperature and concentration of dicofol.

Table VII

Dicofol and DDTr Partition Coefficients
Distributions Between n-Heptane and Raffinate Solvents

| Solvent | Parts Solvent | Parts Water | Parts n-Heptane | Parts Dicofol | Partition Coeff. Dicofol | Partition Coeff. o,p'-DDE | Partition Coeff. p,p'-Cl-DDT |
|---|---|---|---|---|---|---|---|
| Acetic Acid-Methanol | 3.5<br>31.5 | 0.0 | 30 | 15 | 0.33 | 1.11 | 1.40 |
| Acetone | 30 | 3.4 | 30 | 2.25 | 3.40 | 6.43 | 11.14 |
| 1,3-Butanediol | 20 | 0.0 | 30.1 | 7.6 | 0.15 | 6.02 | 6.15 |
| 1,3-Butanediol | 10 | 1.1 | 30 | 4 | 0.84 | 26.60 | 35.32 |
| Ethylene Glycol | 25 | 0.0 | 30 | 4 | 1.20 | 121.40 | 79.49 |
| Formic Acid | 42.99 | 4.51 | 40 | 2.6 | 28.17 | 191.98 | 254.77 |
| Formic Acid-Methanol | 3.17<br>31.5 | 0.33 | 30 | 15 | 0.31 | 1.49 | 1.99 |
| 2,5-Hexanediol | 14.5 | 0.0 | 30.2 | 4.0 | 0.21 | 3.86 | 5.24 |
| 4-Hydroxy-3-methyl-2-butanone | 24 | 3.1 | 30.1 | 4.0 | 0.15 | 2.85 | 4.78 |
| Isopropanol | 30 | 7.7 | 30 | 2.45 | 1.50 | 2.46 | 3.40 |
| Propionic Acid | 20 | 6.5 | 20 | 5 | 6.60 | 21.82 | 38.92 |

Table VIII

Dicofol and DDTr Distribution Coefficients
Distributions Between aqueous Ethanol and Extraction Solvents

| Solvent | Parts Solvent | Parts Water | Parts Ethanol | Parts Dicofol | Partition Coeff. Dicofol | Partition Coeff. o,p'-DDE | Partition Coeff. p,p'-Cl-DDT |
|---|---|---|---|---|---|---|---|
| Cyclohexene | 40 | 11.5 | 29.6 | 8.9 | 8.3 | 25 | 50 |
| 2,4,4-Trimethyl-1-pentene | 40 | 11.5 | 29.6 | 8.9 | 3.82 | 14.0 | 27.2 |
| n-Heptane | 30 | 11.5 | 29.6 | 8.9 | 1.88 | 9.31 | 16.3 |
| Ligroine (b.p. 90-110°C) | 40 | 11.5 | 29.6 | 8.9 | 2.22 | 10.8 | 20.7 |
| Petroleum Ether (b.p. 37-57°C) | 30 | 11.5 | 29.6 | 8.9 | 2.04 | 7.89 | 12.3 |

Table IX

| Results of Extractions with Various Concentrations of Aqueous Ethanol. Distribution Coeff. & Selectivities using Heptane at 23 °C | | | | | | | |
|---|---|---|---|---|---|---|---|
| Upper Layer | Aqueous Ethanol | Conc. of Dicofol | o,p'-p,p' dicofol D.C. | o,p'-DDE | | p,p' Cl-DDT | |
| | | | | D.C. | Sel. | D.C. | Sel. |
| Heptane | 95% | 30% | MISCIBLE | | | | |
| Heptane | 90% | 30% | 0.76 | 1.08 | 1.42 | 0.91 | 1.20 |
| Heptane | 87% | 30% | 0.63 | 1.28 | 2.01 | 1.48 | 2.33 |
| Heptane | 85% | 30% | 0.74 | 2.06 | 2.78 | 1.65 | 2.22 |

Table X

Dicofol and DDTr Distribution Coefficient
Distribution Between Aqueous Methanol and Extraction Solvents

| Solvent | Parts Solvent | Parts Water | Parts Methanol | Parts Dicofol | Partition Coeff. Dicofol | Partition Coeff. o,p'-DDE | Partition Coeff. p,p'-Cl-DDT |
|---|---|---|---|---|---|---|---|
| Cyclohexane | 40 | 3.5 | 31.5 | 15 | 1.00 | 4.66 | 5.48 |
| Cyclohexane | 40 | 5.25 | 29.75 | 15 | 4.17 | 20 | 50 |
| Cyclohexane | 40 | 3.5 | 31.5 | 15 | 1.89 | 5.88 | 10 |
| Decalin | 40 | 3.5 | 31.5 | 15 | 1.04 | 8.3 | 20 |
| 2,4,4-trimethyl-1-pentene | 40 | 5.25 | 29.75 | 15 | 1.33 | 6.43 | 10.0 |
| 2,4,4-trimethyl-1-pentene | 40 | 3.5 | 31.5 | 15 | 0.77 | 3.00 | 4.93 |
| 2,4,4-trimethyl-1-pentene | 40 | 1.75 | 33.25 | 15 | 0.64 | 0.39 | 1.88 |
| n-Eicosane/ n-Heptane | 15<br>15 | 3.5 | 31.5 | 15 | 0.22 | 2.45 | 4.04 |
| 1-Eicosane/ n-Heptane | 25.1<br>5 | 3.55 | 31.94 | 15.21 | 0.28 | 3.08 | 5.44 |

Table X (cont'd)

Dicofol and DDTr Distribution Coefficient
Distribution Between Aqueous Methanol and Extraction Solvents

| Solvent | Parts Solvent | Parts Water | Parts Methanol | Parts Dicofol | Partition Coeff. Dicofol | Partition Coeff. o,p'-DDE | Partition Coeff. p,p'-Cl-DDT |
|---|---|---|---|---|---|---|---|
| Mixed Heptanes[1] | 40 | 3.5 | 31.5 | 15 | 0.40 | 3.79 | 9.48 |
| n-Hexadecane | 30 | 3.5 | 31.5 | 15 | 0.41 | 5.31 | 9.75 |
| Laktane[2] | 40 | 3.5 | 31.5 | 15 | 1.04 | 3.53 | 2.94 |
| Ligroine (b.p. 90-110°C) | 30 | 5.25 | 29.75 | 15 | 0.60 | 4.82 | 6.47 |
| Ligroine (b.p. 90-110°C) | 30 | 3.5 | 31.5 | 15 | 0.34 | 2.75 | 3.69 |
| Ligroine (b.p. 90-110°C) | 30 | 1.75 | 33.25 | 15 | 0.29 | 1.82 | 2.41 |
| 4-methyl-cyclo-hexene | 40 | 3.5 | 31.5 | 15 | 1.69 | 5.88 | 11.1 |
| Octenes (-1 and -2)[3] | 30 | 3.5 | 31.5 | 15 | 0.84 | 3.82 | 5.95 |
| 1-Pentadecene | 19.6 | 3.5 | 31.5 | 15 | 0.41 | 4.30 | 6.53 |
| 2-Pentene | 24.2 | 1.4 | 12.6 | 6 | 1.06 | 3.41 | 4.64 |

Table X (cont'd)

Dicofol and DDTr Distribution Coefficient
Distribution Between Aqueous Methanol and Extraction Solvents

| Solvent | Parts Solvent | Parts Water | Parts Methanol | Parts Dicofol | Partition Coeff. Dicofol | Partition Coeff. o,p'-DDE | Partition Coeff. p,p'-Cl-DDT |
|---|---|---|---|---|---|---|---|
| Petroleum ether (b.p. 37-57°C) | 30 | 5.25 | 29.75 | 15 | 0.49 | 4.22 | 6.69 |
| Petroleum ether (b.p. 37-57°C) | 30 | 3.5 | 31.5 | 15 | 0.34 | 1.69 | 2.20 |
| Petroleum ether (b.p. 37-57°C) | 30 | 1.75 | 33.25 | 15 | 0.33 | 1.37 | 1.77 |
| Toluene | 36 | 8.55 | 36.45 | 5.0 | 7.45 | 34.2 | 59.9 |
| Toluene/ n-Heptane | 20<br>20 | 3.5 | 31.5 | 15 | 1.27 | 3.01 | 3.47 |
| 2,2,4-tri-methylpentane | 40 | 3.5 | 31.5 | 15 | 0.27 | 2.06 | 2.24 |

1 Purchased from Union Oil Co. as "Heptane 1483", b.p. range 92.3° to 96.0°C, composed primarily of $C_7$ saturated hydrocarbons.

2 Laktane is a commercial hydrocarbon mixture containing approximately 30% toluene.

3 Practical grade from Eastman Kodak.

## Table XI

**Distribution of Dicofol and DDTr's**
**Effects of Temperature and Concentration**
**(Aqueous Methanol and n-Heptane System)**

| Solvent | Parts Solvent | Parts Water | Parts Methanol | Parts Dicofol | Temper. °C | Partition Coeff. Dicofol | Partition Coeff. o,p'-DDE | Partition Coeff. p,p'-Cl-DDT |
|---|---|---|---|---|---|---|---|---|
| n-Heptane | 40 | 4.5 | 40.5 | 5 | 23 | 0.32 | 3.91 | 7.52 |
| n-Heptane | 40 | 3.5 | 31.5 | 15 | 23 | 0.30 | 2.41 | 3.98 |
| n-Heptane | 40 | 3 | 27 | 20 | 23 | 0.32 | 2.32 | 2.78 |
| n-Heptane | 40 | 2.5 | 22.5 | 25 | 23 | 0.31 | 1.66 | 2.13 |
| n-Heptane | 40 | 4.5 | 40.5 | 5 | 35 | 0.44 | 4.40 | 8.31 |
| n-Heptane | 40 | 3.5 | 31.5 | 15 | 35 | 0.39 | 2.56 | 3.27 |
| n-Heptane | 40 | 3 | 27 | 20 | 35 | 0.43 | 1.90 | 2.95 |
| n-Heptane | 40 | 2.5 | 22.5 | 25 | 35 | 0.44 | 1.90 | 2.08 |

A laboratory simulation of a continuously operated countercurrent extraction column was accomplished by modeling with an extended series of individual batch extractions. A six stage column having four extraction stages and two backwash stages was found to afford a high degree of DDTr extraction and permit a high level of dicofol recovery (greater than 90%) having increased purity (greater than 92%). The experiment involved batch extractions using 50g of 90% aqueous MeOH/dicofol solutions (40% technical);

50g n-heptane portions; and 20g 90% aqueous MeOH solutions for the backwash. The dicofol feed solutions were mixed with aqueous backwash that had contacted exiting heptane extracts, and were then extracted in counter current manner three times with heptane extracts and then a final time with entering fresh heptane. Results of this analysis are presented in Table XII.

Table XII

| Batch Simulation of Continuous Counter Current Distribution: Four Extraction Stages, Two Backwash Stages at 23° C Analysis of Purified Dicofol Concentrate | | |
|---|---|---|
| Component | Dicofol Feed | Product |
| pp DCBZ | 0.3 | .13 |
| op' Dicofol | 13.0 | 14.3 |
| pp' Dicofol | 70.3 | 77.9 |
| op' DDE | 0.9 | 0.04 |
| pp' DDE | 0.09 | --[1] |
| op' Cl-DDT | 0.20 | -- |
| pp' Cl-DDT | 3.4 | 0.1 |
| % DDTr | 4.6 | 0.14 |
| Sample Weight | 20.0 | 16.87 |
| Dicofol Recovery, % | | 93% |

[1] Not detected.

To establish commercial feasibility a pilot plant scale demonstration was conducted. At the conclusion of the pilot trials, a "production run" was made to demonstrate extended operations. More than 45 kg (one hundred pounds) of dicofol was purified in this run. Approximately 98% of the dicofol was recovered.

Pilot plant production run purifications of dicofol were done in a Karr reciprocatory plate extraction column under the conditions listed below in Table XIII. As the feed solution passes through the column, it initimately mixes with a non-polar hydrocarbon solvent. By introducing the feed at the midpoint of the column, the backwash stream recaptures dicofol that has transferred to the hydrocarbon solvent phase.

Table XIII

| Dicofol Purification Production Run Conditions | |
|---|---|
| Colummn diameter | 2.54 cm |
| Weight concentration of dicofol | 32.57% |
| Feed rate | 87 g/minute |
| Solvent rate | 90 g/minute |
| Backwash rate | 55 g/minute |
| Total column flow | 35042 1/hr/$m^2$ (860 gal/hr./$ft.^2$) |
| Temperature | 35° C |
| Column agitator strokes | 50/minute |

A detailed analysis of the dicofol made in the "production run" is presented in Table XIV where it is contrasted with the dicofol technical before extraction. DDTr's were reduced from about 5.5% to less than 0.1% by removal of DDE and Cl-DDT isomers. The extent of removal was greater than the removal achieved in laboratory modeling trials (4.6% reduced to 0.15%). A significant increase in active ingredient content (from about 85% to about 96% was achieved). No significant change in the proportions of the two dicofol isomers occurs.

Table XIV

Purification of Dicofol in Pilot Plant

| Component | Before[1] | After[2] |
|---|---|---|
| **Active Ingredient** | | |
| p,p'-Dicofol | 70.7% | 80.7% |
| o,p'-Dicofol | 14.0% | 15.4% |
| Total Dicofol | 84.7% | 96.1% |
| **DDTr Impurities** | | |
| p,p'-DDT | <.01% | < 5 ppm |
| o,p'-DDT | <.01% | < 5 ppm |
| p,p'-DDE | 0.1% | < 5 ppm |
| o,p'-DDE | 1.3% | 84 ppm |
| p,p'-DDD | <.01% | < 5 ppm |
| o,p'-DDD | <.01% | < 5 ppm |
| p,p'-ClDDT | 3.9% | 15 ppm |
| o,p'-ClDDT | 0.2% | 90 ppm |
| Total DDTr's | 5.5% | 190-225 ppm (0.02%) |
| **Non-DDTr Impurities** | | |
| p,p'-DCBP | 0.3% | 0.4% |
| o,p'-DCBP | 0.5% | 0.2% |
| p,p'-DCBZ | 0.2% | 0.14% |
| o,p'-DCBZ | 1.0% | 1.0% |
| Total Non-DDTr's | 2.0% | 1.7% |
| **Unidentified Ingredients** | 7.8% | 1.9%[3] |
| Total Components | 100.0 | 100.0 |

[1] Composition of dicofol technical used in pilot trials.
[2] Limit of detection for DDTr's = 5 ppm.
[3] Polar materials unrelated to DDT.

## Claims

1. A process for reducing the amount of DDTr impurities in 1,1-bis(chlorophenyl)-2,2,2-trichloroethanol containing them, which comprises, at a temperature of from -5° C to 100° C:
   (a) forming a solution of 1,1-bis(chlorophenyl)-2,2,2-trichloroethanol containing DDTr impurities in

water-miscible solvent to obtain a solution having a 1,1-bis(chlorophenyl)-2,2,2-trichloroethanol concentration of from 1% to 60% by weight; and
(b) extracting the solution with solvent comprising ($C_4$-$C_{20}$)alkane, ($C_4$-$C_{20}$)alkene, ($C_6$-$C_{20}$)alkadiene, ($C_6$-$C_{10}$) single ring aromatic, or a mixture thereof; and
(c) separating the resulting phases; and
(d) removing the solvent from the dicofol-containing phase.

**2.** A process as claimed in claim 1 wherein the water-miscible solvent comprises one or more hydroxylic solvent and, optionally, the solution contains water in an amount of up to 50% by weight based on the amount of non-aqueous solvent.

**3.** A process as claimed in claim 2 wherein the hydroxylic solvent comprises ($C_1$-$C_3$) alcohol, ($C_2$-$C_6$) diol, ($C_1$-$C_3$) organic acid or 4-hydroxy-3-methyl-2-butanone, and/or solvent formed in situ from water and solvent, preferably acetone, capable of forming hydroxylic solvent when mixed with water.

**4.** A process as claimed in any preceding claim wherein the extraction is continuous.

**5.** A process as claimed in any preceding claim wherein the solvent in (a) comprises methanol, ethanol, ($C_2$-$C_6$) diol or a mixture thereof, containing up to 30% water.

**6.** A process as claimed in any preceding claim wherein the solution formed in (a) has a 1,1-bis-(chlorophenyl)-2,2,2-trichloroethanol concentration of from 10% to 50% by weight.

**7.** A process as claimed in any preceding claim wherein the solvent in (b) comprises ($C_5$-$C_{16}$)alkane, ($C_5$-$C_{15}$)alkene or a mixture thereof.

**8.** A process as claimed in any preceding claim carried out between 10°C and 60°C, preferably between 20°C and 40°C.

**9.** A process as claimed in any preceding claim wherein methanol or methanol containing from 7% to 13% preferably 9% to 13%, by weight water is employed as the solvent in step (a).

**10.** A process as claimed in any preceding claim wherein mixed heptanes are employed as the solvent in step (b).

**11.** A process for reducing the amount of preparing DDTr impurities in 1,1-bis(chlorophenyl)-2,2,2-trichloroethanol containing them which comprises, at a temperature of from 20°C to 40°C:
(a) forming a solution of 1,1-bis(chlorophenyl)-2,2,2-trichloroethanol containing DDTr impurities in aqueous methanol containing from 9% to 13% by weight, based on the weight of methanol, of water to obtain a solution having a 1,1-bis(chlorophenyl)-2,2,2-trichloroethanol concentration of from 25% to 35% by weight; and
(b) extracting the solution with mixed heptanes; and
(c) separating the resulting phases; and
(d) removing the solvent from the dicofol containing phase to yield 1,1-bis(chlorophenyl)-2,2,2-trichloroethanol having less than 0.1% DDTr's.

**12.** A process as claimed in claim 11 wherein said extraction is carried out by continuous liquid-liquid countercurrent extraction.

## Revendications

**1.** Un procédé pour réduire la quantité d'impuretés de DDTr dans le 1,1-bis(chlorophényl)-2,2,2-trichloroéthanol les contenant, qui comprend, à une température de -5°C à 100°C :
(a) la formation d'une solution de 1,1-bis(chlorophényl)-2,2,2-trichloroéthanol contenant des impuretés de DDTr dans un solvant miscible à l'eau pour obtenir une solution ayant une concentration en 1,1-bis(chlorophényl)-2,2,2-trichloroéthanol de 1 % à 60 % en poids ; et
(b) l'extraction de la solution avec du solvant comprenant un alcane en $C_4$-$C_{20}$, un alcène en $C_4$ à $C_{20}$, un alcoyldiène en $C_6$ à $C_{20}$, un composé aromatique à un seul noyau en $C_6$ à $C_{10}$, ou un

mélange de ceux-ci ; et
(c) la séparation des phases résultantes ; et
(d) l'élimination du solvant à partir de la phase contenant le dicofol.

**2.** Un procédé comme revendiqué dans la revendication 1 dans lequel le solvant miscible à l'eau comprend un ou plusieurs solvants hydroxyliques et, éventuellement, la solution contient de l'eau en une quantité allant jusqu'à 50 % en poids sur la base de la quantité de solvant non aqueux.

**3.** Un procédé comme revendiqué dans la revendication 2, dans lequel le solvant hydroxylique comprend un alcool en $C_1$-$C_3$, un diol en $C_2$ à $C_6$, un acide organique en $C_1$ à $C_3$ ou la 4-hydroxy-3-méthyl-2-butanone, et/ou un solvant formé in situ à partir d'eau et de solvant, de préférence d'acétone, capable de former le solvant hydroxylique lorsqu'il est mélangé à l'eau.

**4.** Un procédé comme revendiqué dans l'une quelconque des revendications précédentes, dans lequel l'extraction est continue.

**5.** Un procédé comme revendiqué dans l'une quelconque des revendications précédentes dans lequel le solvant dans (a) comprend le méthanol, l'éthanol, un diol en $C_2$-$C_6$ ou un mélange de ceux-ci, contenant jusqu'à 30 % d'eau.

**6.** Un procédé comme revendiqué dans l'une quelconque des revendications précédentes, dans lequel la solution formée dans (a) a une concentration en 1,1-bis(chlorophényl)-2,2,2-trichloroéthanol comprise entre 10 et 50 % en poids.

**7.** Un procédé comme revendiqué dans l'une quelconque des revendications précédentes, dans lequel le solvant dans (b) comprend un alcane en $C_5$ à $C_{16}$, un alcène en $C_5$ à $C_{15}$ ou un mélange de ceux-ci.

**8.** Un procédé comme revendiqué dans l'une quelconque des revendications précédentes, mis en oeuvre entre 10°C et 60°C, de préférence entre 20°C et 40°C.

**9.** Un procédé comme revendiqué dans l'une quelconque des revendications précédentes, dans lequel du méthanol, ou du méthanol contenant de 7 à 13 %, de préférence 9 à 13 en poids d'eau, est utilisé comme solvant dans la phase (a).

**10.** Un procédé comme revendiqué dans l'une quelconque des revendications précédentes, dans lequel des heptanes mélangés sont utilisés en tant que solvant dans la phase (b).

**11.** Un procédé pour réduire la quantité d'impuretés préparatoires de DDTr dans 1,1-bis(chlorophényl)-2,2,2-trichloroéthanol les contenant, qui comprend, à une température comprise entre 20°C et 40°C :
(a) la formation d'une solution de 1,1-bis(chlorophényl)-2,2,2-trichloroéthanol contenant des impuretés de DDTr dans du méthanol aqueux contenant de 9 à 13 % en poids, sur la base du poids de méthanol, d'eau pour obtenir une solution ayant une concentration en 1,1-bis(chlorophényl)-2,2,2-trichloroéthanol comprise entre 25 % et 35 % en poids ; et
(b) l'extraction de la solution au moyen d'heptanes mélangés ; et
(c) la séparation des phases résultantes ; et
(d) l'élimination du solvant à partir de la phase contenant le dicofol pour donner 1,1-bis-(chlorophényl)2,2,2-trichloroéthanol ayant moins de 0,1 % de DDTr.

**12.** Un procédé tel que revendiqué dans la revendication 11, dans lequel ladite extraction est mise en oeuvre par extraction continue à contre-courant liquide-liquide.

**Patentansprüche**

**1.** Verfahren zum Herabsetzen der Menge an DDTr-Verunreinigungen in sie enthaltendem 1,1-Bis-(chlorphenyl)-2,2,2-trichlorethanol, bei dem man bei einer Temperatur von -5 bis 100 °C
(a) eine Lösung von DDTr-Verunreinigungen enthaltendem 1,1-Bis(chlorphenyl)-2,2,2-trichlorethanol in einem mit Wasser mischbaren Lösemittel herstellt, um eine Lösung zu erhalten, die eine Konzentration an 1,1-Bis(chlorphenyl)-2,2,2-trichlorethanol von 1 bis 60 Gew.-% aufweist; und

(b) die Lösung mit einem Lösemittel extrahiert, welches ($C_4$-$C_{20}$)-Alkan, ($C_4$-$C_{20}$)-Alken, ($C_6$-$C_{20}$)-Alkadien, ($C_6$-$C_{10}$)-Einfachringaromaten oder ein Gemisch davon enthält; und
(c) die entstehenden Phasen trennt; und
(d) das Lösemittel von der Dicofol enthaltenden Phase entfernt.

**2.** Verfahren nach Anspruch 1, wobei das mit Wasser mischbare Lösemittel ein oder mehrere hydroxische Lösemittel enthält und die Lösung wahlweise Wasser in einer Menge von bis zu 50 Gew.-%, bezogen auf die Menge des nichtwäßrigen Lösemittels, enthält.

**3.** Verfahren nach Anspruch 2, wobei das hydroxische Lösemittel ($C_1$-$C_3$)-Alkohol, ($C_2$-$C_6$)-Diol, ($C_1$-$C_3$)-organische Säuren oder 4-Hydroxy-3-methyl-2-butanon und/oder ein Lösemittel enthält, welches in situ aus Wasser und einem Lösemittel, bevorzugt Aceton, gebildet wurde, welches beim Mischen mit Wasser zum Bilden eines hydroxischen Lösemittels befähigt ist.

**4.** Verfahren nach einem der vorhergehenden Ansprüche, wobei man die Extraktion kontinuierlich durchführt.

**5.** Verfahren nach einem der vorhergehenden Ansprüche, wobei das Lösemittel in (a) Methanol, Ethanol, ($C_2$-$C_6$)-Diol oder ein Gemisch davon enthält, welches bis zu 30 % Wasser enthält.

**6.** Verfahren nach einem der vorhergehenden Ansprüche, wobei die in (a) gebildete Lösung eine Konzentration an 1,1-Bis-(chlorphenyl)-2,2,2-trichlorethanol von 10 bis 50 Gew.-% aufweist.

**7.** Verfahren nach einem der vorhergehenden Ansprüche, wobei das Lösemittel in (b) ($C_5$-$C_6$)-Alkan, ($C_5$-$C_{15}$)-Alken oder ein Gemisch davon enthält.

**8.** Verfahren nach einem der vorhergehenden Ansprüche, durchgeführt zwischen 10 und 60 °C, bevorzugt zwischen 20 und 40 °C.

**9.** Verfahren nach einem der vorhergehenden Ansprüche, wobei man als Lösemittel in Schritt (a) Methanol einsetzt oder Methanol mit einem Gehalt von 7 bis 13 Gew.-% Wasser, bevorzugt 9 bis 13 Gew.-%.

**10.** Verfahren nach einem der vorhergehenden Ansprüche, wobei man als Lösemittel in Schritt (b) gemischte Heptane einsetzt.

**11.** Verfahren zum Herabsetzen der Menge beim Herstellen von DDTr-Verunreinigungen in sie enthaltendem 1,1-Bis (chlorphenyl)-2,2,2-trichlorethanol, bei dem man bei einer Temperatur von 20 bis 40 °C
(a) eine Lösung von DDTr-Verunreinigungen enthaltendem 1,1-Bis(chlorphenyl)-2,2,2-trichlorethanol in wäßrigem Methanol hergestellt, welches bezogen auf das Gewicht des Methanols 9 bis 13 Gew.-% Wasser enthält, um eine Lösung zu erhalten, die eine Konzentration an 1,1-Bis(chlorphenyl)-2,2,2-trichlorethanol von 25 bis 35 Gew.-% aufweist; und
(b) die Lösung mit gemischten Heptanen extrahiert; und
(c) die entstehenden Phasen trennt; und
(d) das Lösemittel aus der Dicofol enthaltenden Phase zum Gewinnen von 1,1-Bis(chlorphenyl)-2,2,2-trichlorethanol entfernt, welches weniger als 0,1 % DDTr aufweist.

**12.** Verfahren nach Anspruch 11, wobei man die Extraktion als kontinuierliche Flüssig/Flüssig-Gegenstrom-Extraktion durchführt.